# EUROPÄISCHE PATENTSCHRIFT

(11) **EP 1 523 490 B1**
(45) Veröffentlichungstag und Bekanntmachung des Hinweises auf die Patenterteilung: **23.12.2009**
(21) Anmeldenummer: 03729788.4
(22) Anmeldetag: 02.07.2003
(51) Int. Cl.: C07J 73/00

(54) **VERFAHREN ZUR EINFUEHRUNG EINER 1,2-DOPPELBINDUNG BEI 3-OXO-4 -AZASTEROIDVERBINDUNGEN**
METHOD FOR INTRODUCING A 1,2 DOUBLE BOND INTO 3-OXO-4-AZASTEROID COMPOUNDS
PROCEDE D'INTRODUCTION D'UNE DOUBLE LIAISON 1,2 DANS DES COMPOSES 3-OXO-4-AZASTEROIDES

(30) Priorität: 16.07.2002 CH 124202; 08.08.2002 CH 137502; 08.01.2003 CH 15032003
(43) Veröffentlichungstag der Anmeldung: 20.04.2005
(73) Patentinhaber: Siegfried Generics International AG, 4800 Zofingen (CH)
(72) Erfinder: SCHÄRER, Norbert, CH-5036 Oberentfelden (CH); WEBER, Beat, CH-4800 Zofingen (CH); MÜLLER, Beat, W., CH-4106 Therwil/BL (CH)
(74) Vertreter: Braun, André jr.
(86) Internationale Anmeldenummer: PCT/CH2003/000435
(87) Internationale Veröffentlichungsnummer: WO 2004/007523

(56) Entgegenhaltungen:
- EP-A- 0 298 652
- EP-A- 0 428 366
- US-A- 5 710 342
- RASMUSSON G H ET AL: "AZASTEROIDS: STRUCTURE-ACTIVITY RELATIONSHIPS FOR INHIBITION OF 5ALPHA-REDUCTASE AND OF ANDROGEN RECEPTOR BINDING" JOURNAL OF MEDICINAL CHEMISTRY, AMERICAN CHEMICAL SOCIETY. WASHINGTON, US, Bd. 29, Nr. 11, 1. November 1986 (1986-11-01), Seiten 2298-2315, XP000568779 ISSN: 0022-2623
- BHATTACHARYA APURBA ET AL: "Silylation-mediated oxidation of 4-aza-3-ketosteroids with DDQ proceeds via DDQ-substrate adducts" JOURNAL OF THE AMERICAN CHEMICAL SOCIETY, AMERICAN CHEMICAL SOCIETY, WASHINGTON, DC, US, Bd. 110, 1988, Seiten 3318-3319, XP002179347 ISSN: 0002-7863
- J. TSUJI ET AL: "PALLADIUM CATALYZED PREPARATION OR lpha.-ALLYL ETSRS AND alpha,beta-UNSATURATED ESTERS FROM SATURATED ESTERS VIA THEIR KETENE SILYL ACETALS" TETRAHEDRON LETTERS., Bd. 25, Nr. 42, 1984, Seiten 4783-4786, XP002226639 ELSEVIER SCIENCE PUBLISHERS, AMSTERDAM., NL ISSN: 0040-4039 in der Anmeldung erwähnt

## Beschreibung

Die vorliegende Erfindung betrifft ein Verfahren zur Einführung einer 1,2-Doppelbindung bei 3-Oxo-4-azasteroiden durch Dehydrierung von in 1,2-Stellung gesättigten 3-Oxo-4-azasteroiden, insbesondere durch Dehydrierung von 17βsubstituierten 3-Oxo-4-azasteroiden, zur Herstellung der entsprechenden 17β-substituierten 3-Oxo-4-azasteroide, welche in der 1,2-Stellung eine Doppelbindung aufweisen.

Aus EP 0 155 096 ist es bekannt, 17β-substituierte 4-Aza-5-alfa-Androstane mit einer 1,2-Doppelbindung herzustellen, indem man die entsprechende 1,2-Dihydroverbindung mittels Benzol-Seleninsäureanhydrid oxydiert. Weitere Verfahren zur Einführung einer 1,2-Doppelbindung bei 17β-substituierte 4-Aza-5-alfa-androstanen sind beispielsweise auch in EP 0 298 652, EP 0 428 366 und EP 0 473 225, beschrieben. 17β-substituierte 4-aza-5-alfa-Androstane mit einer 1,2-Doppelbindung sind vielseitig eingesetzte pharmazeutisch wirksame Verbindungen. Von Bedeutung ist beispielsweise die Verbindung 17β-(N-tert.-Butylcarbamoyl)-4-aza-androst-1-en-3-on (Finasterid), welche beispielsweise als 5-alfa-Reduktase-Hemmer zur Behandlung von begniner Prostata-Hyperplasie bzw. von *alopecia androgenetica* verwendet wird. Von Bedeutung ist beispielsweise auch 17β-{N-[2,5-bis(trifluoromethyl)phenyl]}-4-aza-androst-1-en-3-on (Dutasterid). Die bekannten Verfahren zur Herstellung dieser Verbindungen haben spezifische Nachteile, so dass ein Bedürfnis für verbesserte alternative Verfahren besteht.

Die vorliegende Erfindung betrifft solch ein alternatives Herstellungsverfahren.

Die vorliegende Erfindung ist in den Patentansprüchen definiert. Die vorliegende Erfindung betrifft ein Verfahren zur Herstellung von 17β-substituierten 4-Aza-androst-1-en-3-on-Verbindungen der allgemeinen Formel (I): 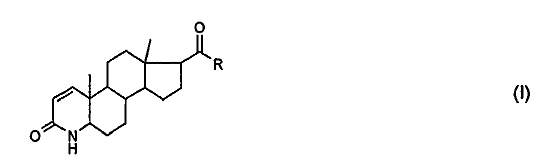 worin
- R: Hydroxyl, gegebenenfalls substituiertes, lineares oder verzweigtes (C₁-C₁₂) -Alkyl oder (C₁-C₁₂)-Alkenyl; Phenyl oder Benzyl; einen Rest -OR₁, oder einen Rest -NHR₁, oder einen Rest -NR₁R₂;
- R₁: Wasserstoff, gegebenenfalls substituiertes, lineares oder verzweigtes (C₁-C₁₂)-Alkyl oder (C₁-C₁₂)-Alkenyl, oder gegebenenfalls substituiertes Phenyl;
- R₂: Wasserstoff, Methyl, Ethyl oder Propyl; oder
-NR₁R₂ einen 5- oder 6-gliedrigen heterocyclischen Ring, und für R = Hydroxyl auch ein pharmazeutisch zugelassenes Salz davon,
bedeuten, **dadurch gekennzeichnet, dass** man
(A) in die 3-Keto-4-aza-Gruppierung (Lactamgruppierung) einer Verbindung der allgemeinen Formel (II): 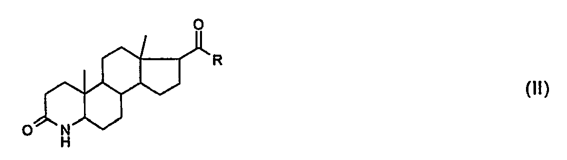 Schutzgruppen einführt, so dass eine Verbindung der allgemeinen Formel (III) entsteht: 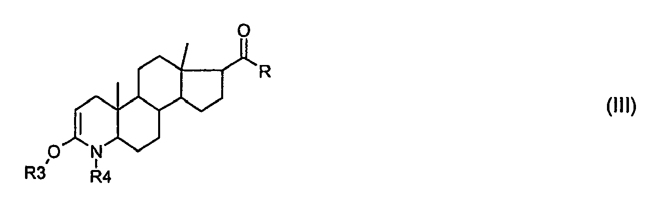 worin
   - R₃: Trialkylsilyl, oder zusammen mit R₄ den Rest -C(O)-C(O)- oder -C(O)-Y-C(O)-;
   - R₄: Alkyloxycarbonyl oder Phenyloxycarbonyl, vorzugsweise Boc (= tert.-Butyloxycarbonyl); oder Trialkylsilyl, oder zusammen mit R₃ den Rest -C(O)-C(O)- oder -C(O)- Y-C(O)-;
   - Y: -[C(R₅)(R₆)]ₙ-, oder -CH(R₅)=CH(R₆)-, oder ortho- Phenylen;
   - R₅ und R₆: unabhängig voneinander Wasserstoff, lineares oder verzweigtes (C₁₋₈)-Alkyl oder Alkenyl, gegebenenfalls substituiertes Phenyl oder Benzyl; und
   - n: eine ganze Zahl von 1 bis 4,
   bedeuten;
   und worin für den Fall, dass R Hydroxyl bedeutet, diese gegebenenfalls mit einer Schutzgruppe reagiert hat;
(B) die [gemäss Schritt (A)] erhaltene Verbindung in Gegenwart (i) eines Dehydrierungskatalysators, der hierin definiert wird, und in Gegenwart von (ii) gegebenenfalls substituiertem Benzochinon, Allylmethylcarbonat, Allylethylcarbonat und/oder Allylpropylcarbonat, umsetzt, wobei die Δ¹-Doppelbindung in 1/2-Stellung eingeführt wird, und
(C) die Schutzgruppen R₃ und R₄ entfernt und für R = Hydroxyl, die erhaltene Verbindung gegebenenfalls in ein Salz umwandelt.

R bedeutet vorzugsweise lineares oder verzweigtes (C₁-C₆)-Alkyl, vorzugsweise Methyl, Ethyl, Propyl oder n-Butyl, sec.-Butyl oder tert.-Butyl, vorzugsweise tert.-Butyl; oder einen Rest -OR₁, oder ein Rest -NHR₁, oder ein Rest -NR₁R₂. Bevorzugt ist der Rest -NHR₁.

Bedeutet R Hydroxyl (bzw. der Rest -C(O)R bedeutet Carboxyl), so kann erfindungsgemäss auch ein pharmazeutisch zugelassenes Salz der Verbindung der Formel (I) hergestellt werden, vorzugsweise ein Alkalisalz, ein Erdalkalisalz oder ein Ammoniumsalz, vorzugsweise ein Salz von Natrium, Kalium oder Ammonium, vorzugsweise ein Salz von Natrium oder Kalium.

R₁ bedeutet vorzugsweise lineares oder verzweigtes (C₁-C₆)-Alkyl, oder gegebenenfalls substituiertes Phenyl. R₁ als (C₁-C₆)-Alkyl bedeutet vorzugsweise Methyl, Ethyl, Propyl, n-Butyl, sec.-Butyl oder tert.-Butyl, vorzugsweise tert.-Butyl. R₁ als gegebenenfalls substituiertes Phenyl bedeutet vorzugsweise Mono(trifluoromethyl)phenyl oder Bis(trifluoromethyl)phenyl, vorzugsweise 2,5-Bis(trifluoromethyl)phenyl.

Im Rest -NR₁R₂ bedeutet R₂ vorzugsweise Methyl.

Der Substituent -NR₁R₂ als 5- oder 6-gliedriger heterocyclischer Ring bedeutet vorzugsweise einen Rest von Piperidin oder Pyrrolidin.

Bevorzugt ist der Substitutent -NHR₁, worin R₁ tert.-Butyl oder 2,5-Bis(trifluoromethyl)phenyl bedeutet.

R₃ bedeutet vorzugsweise Trimethylsilyl, oder zusammen mit R₄ den Rest -C(O)-C(O)- oder -C(O)-Y-C(O)-.

R₄ bedeutet vorzugsweise Boc, Trimethylsilyl, oder zusammen mit R₃ den Rest -C(O)-C(O)- oder -C(O)-Y-C(O)-. Vorzugsweise bedeutet R₄ Boc oder zusammen mit R₃ den Rest -C(O)-C(O)- oder -C(O)-Y-C(O)-.

R₄ als Alkyloxycarbonyl bedeutet vorzugsweise Isobutyloxycarbonyl, tert.-Butyloxycarbonyl, tert.-Amyloxycarbonyl, Cyclobutyloxycarbonyl, 1-Methylcylobutyloxycarbonyl, Cyclopentyloxycarbonyl, Cyclohexyloxycarbonyl, 1-Methylcyclohexyl, vorzugsweise tert.-Butyloxycarbonyl.

R₅ und R₆ bedeuten unabhängig voneinander vorzugsweise Wasserstoff, lineares oder verzweigtes (C₁₋₄)-Alkyl, oder Phenyl, vorzugsweise Wasserstoff, Methyl, Ethyl oder Propyl oder Phenyl.

n bedeutet vorzugsweise 1 oder 2, vorzugsweise 1.

Vorzugsweise bedeutet Y den Rest -CH(R₅)- oder ortho-Phenylen, vorzugsweise Methylen.

Zur Einführung der Schutzgruppe Trialkylsilyl, d.i. zur Silylierung der NH-Gruppe und/oder des Sauerstoffatoms bzw. der OH-Gruppe [gemäss Schritt (A)] verwendet man vorzugsweise ein (Alkyl)₃Si(Halogen), z.B. (CH₃)₃SiCl, oder Bistrimethylsilyltrihalogenacetamid, Bistrimethylsilylacetamid, Hexamethyldisilazan und/oder Bistrimethylharnstoff, vorzugsweise Bistrimethylsilyltrifluoroacetamid, oder ein Trialkylsilyl-trifluoromethansulfonat, vorzugsweise Trimethylsilyl-trifluoromethansulfonat. Die Reaktionsbedingungen für die Silylierung sind aus EP 0 473 226 bekannt.

Für die Einführung einer Schutzgruppe, worin R₃ zusammen mit R₄ den Rest -C(O)-C(O)- oder -C(O)-Y-C(O)- bedeuten, setzt man die Verbindung der allgemeinen Formel (II) bzw. die Laktamgruppierung [gemäss Schritt (A)] mit Oxalylchlorid (Oxalsäurechlorid) oder Malonylchlorid (Malonsäurechlorid) um, wobei Oxalylchlorid bevorzugt ist. Die Reaktionsbedingungen für die Umsetzung mit Oxalylchorid sind EP 0 428 366 bekannt und sind für die Umsetzung mit Malonylchlorid oder analog reagierender Verbindungen in analoger Weise anzuwenden.

Für die Einführung einer Schutzgruppe, worin R₄ Alkyloxycarbonyl, z.B. tert.-Butyloxycarbonyl (Boc) bedeutet, geht man in an sich bekannter Weise vor, indem man die Verbindung der allgemeinen Formel (II) z.B. mit Boc-Anhydrid (Boc-O-Boc) {[(CH₃)₃C-O-C(O)]₂-O} oder mit Boc-Carbamat [(CH₃)₃C-O-C(O)-N(C₁₋₄-Alkyl)₂], umsetzt. Dabei steht hier Boc stellvertretend für die anderen gleich reagierenden Verbindungen, das heisst Verbindungen, worin der tert.-Butylrest ersetzt ist durch einen andern gleich reagierenden Rest, wie beispielsweise die genannten Reste tert.-Amyl, Cyclobutyl, Cyclopentyl oder Cyclohexyl. Solche analogen Reaktionen sind zahlreich in der Fachliteratur beschrieben. Bedeutet R₃ Trialklylsilyl und R₄ Boc, so führt man zuerst die Schutzgruppe Boc ein und silyliert anschliessend.

In Schritt (B) wird die gemäss Schritt (A) erhaltene Verbindung in Gegenwart (i) eines Dehydrierungskatalysators und in Gegenwart von (ii) gegebenenfalls substituiertem Benzochinon, Allylmethylcarbonat, Allylethylcarbonat und/oder Allylpropylcarbonat, umgesetzt, wobei die Δ¹-Doppelbindung in 1-/2-Stellung eingeführt wird. Der Dehydrierungskatalysator ist ausgewählt aus Verbindungen (Salze und Komplexe) von Eisen (Fe), Ruthenium (Ru) und Osmium (Os); Cobalt (Co), Rhodium (Rh), und Iridium (Ir); Nickel (Ni), Palladium (Pd) und Platin (Pt) sowie der Gruppe IB, d.h. von Kupfer (Cu), Silber (Ag) und Gold (Au). Bevorzugt sind Verbindungen der obengenannten Metalle, die von der Gruppe VIII des Periodensystems sind. Bevorzugt sind insbesondere Verbindungen bzw. Dehydrierungskatalysatoren auf der Basis von Rhodium (Rh), Palladium (Pd) und Platin (Pt). Bevorzugt sind Palladiumverbindungen. Beispiele für solche Palladiumverbindungen sind: Pd(0)-Verbindungen wie Tris(dibenzylidenaceton)diPalladium-ChloroformKomplex und Pd(II)-Verbindungen wie PdCl₂, Pd(dppe)₂, [dppe = bis-(1,2-biphenylphosphino)ethan], Pd(dppe)Cl₂, Pd(OAc)₂, Pd(dppe)(OAc)₂, π-Allyl-Pd-Komplexe, vorzugsweise π-Allyl-Pd-chlorid Dimer. Bevorzugt sind Pd(0)-Verbindungen, insbesondere Tris(dibenzylidenaceton)diPalladium Chloroform-Komplex. Diese Verbindungen, bzw. Salze und Komplexe, sind an sich bekannt und in der Literatur beschrieben worden.

Zur termischen Stabilisierung des Palladium-Komplexes kann ein zusätzlicher Komplexbildner wie 2,2'-Bipyridyl oder 1,10-Phenanthrolin eingesetzt werden, vorzugsweise 2,2'-Bipyridyl.

Erklärungshalber kann zum Mechanismus der Katalyse angeführt werden, dass eine Pd-Spezies am C-Atom in 2-Stellung unter Abspaltung der Sauerstoff-Schutzgruppe [z.B. der -Si(CH₃)₃-Gruppe] addiert. Eine anschliessende beta-Wasserstoff-Abspaltung am C-Atom in 1-Stellung führt zur gewünschten Δ¹-Doppelbindung in 1-/2-Stellung, und setzt eine weitere Palladium-Spezies frei, die in den katalytischen Zyklus zurück geführt wird. Hinweise für diesen Reaktionsmechanismus finden sich in Tetrahydron Letters, Seite 4783, (1984). Die vorliegende Erfindung ist aber nicht an diese Erklärung gebunden.

Als Chinon kann man auch ein substituiertes Chinon verwenden, beispielsweise ein durch C₁₋₄-Alkyl, Halogen, Cyano oder Nitro substituiertes Chinon. Solche Chinone sind an sich bekannt.

In Schritt (C) wird dann die erhaltene Verbindung in die Verbindung der Formel (I) umgewandelt, indem man die eingeführten Schutzgruppen entfernt. Dies geschieht vorzugsweise durch Behandlung mit einer geeigneten Säure, beispielsweise mit Ameisensäure, Essigsäure und/oder Trifluoressigsäure, vorzugsweise mit Ameisensäure. Anschliessend kann man gegebenenfalls die erhaltene Verbindung in an sich bekannter Weise in ein pharmazeutisch verwendbares Salz überführen (für R = Hydroxyl).

Vorzugsweise kristallisiert man die erhaltenen Verbindung um. Diese Umkristallisation kann in apolaren Lösungsmitteln wie Benzin, Heptan, Hexan und Toluol, vorzugsweise Toluol, durchgeführt werden. Bei der Verbindung der Formel (I) handelt es sich insbesondere um die eingangs erwähnte Verbindung 17β-(N-tert.-Butylcarbamoyl)-4-aza-androst-1-en-3-on (Finasterid), welche in zwei polymorphen Formen, nämlich polymorphe Form I und polymorphe Form II, auftritt, wobei Form I bevorzugt ist. Form I entsteht beispielsweise bei der Umkristallisation von erfindungsgemäss erhaltenem rohem Finasterid aus einer gesättigten Lösung aus Toluol (etwa ein Teil rohes Finasterid in etwa sechs Teilen Toluol) beim Abkühlen auf etwa 25°C. Die polymorphe Form II entsteht beispielsweise bei der Umkristallisation von erfindungsgemäss erhaltenem rohem Finasterid aus einer Lösung aus Toluol (etwa ein Teil rohes Finasterid in etwa sechs Teilen Toluol) beim Abkühlen auf etwa 0°C.

Die Eigenschaften von 17β-{N-[2,5-bis(trifluoromethyl)phenyl]}-4-aza-androst-1-en-3-on (Dutasterid) sind aus der Literatur bekannt.

Für das beschriebene Verfahren mit den Schritten (A)-(C) können als Lösungsmittel zahlreiche organische wasserfreie Verbindungen verwendet werden, wie beispielsweise Toluol, Benzin, Hexan, Heptan, tert.-Butylalkohol, Diethylether, Aceton, Benzol, Dioxan, Tetrahydrofuran, Chloroform, Dimethylformamid oder Pyridin. Die folgenden Beispiele erläutern die Erfindung.

### Beispiel 1 (Substitution von Dihydrofinasterid mit Boc am Stickstoffatom der 3-Keto-4-aza-Gruppierung)

10 g (26,7 mMol) Dihydrofinasterid werden in Tetrahydrofuran (THF) vorgelegt und auf -78°C abgekühlt. Zur erhaltenen Suspension werden 15 ml (30 mMol) Lithium-diisopropylamid-Lösung (LDA-Lösung) zudosiert und die klare Lösung ca. 30 Minuten gerührt. Dann wird eine Lösung aus 6,7 g (30 mMol) Boc-Anhydrid in THF zudosiert. Die Lösung lässt man nun auf Raumtemperatur (RT) erwärmen. Nach der üblichen Aufarbeitung erhält man ein feuchtes gelbes Pulver, welches über Nacht im Trockenschrank gelagert und direkt in Beispiel 2 eingesetzt wird.

### Beispiel 2 (Silylierung der in Beispiel 1 hergestellten Verbindung)

1 g (2,1 mMol) 4-Boc-Dihydrofinasterid werden in THF gelöst. Zur klaren gelben Lösung gibt man unter Methanol-EisKühlung 2,3 ml (4,6 mMol) LDA-Lösung. Die Suspension wird etwa 45 Minuten gerührt, worauf 0.46 g (4,2 mMol) Trimethylchlorsilan (TMSCl) bei 18-20°C zugetropft wird. Die klare Lösung wird eingeengt und der Rückstand in Heptan aufgenommen. Nach der Filtration wird das Filtrat soweit möglich eingeengt und das erhaltene honigbraune Öl in der folgenden Stufe (Beispiel 3 und Beispiel 5) eingesetzt.

### Beispiel 3 (Einführung der Δ¹-Doppelbindung zu 4-Benzyloxycarbonylfinasterid)

0.145 g (0.65 mMol) Palladiumacetat werden mit 0.07 g (0.65 mMol) Benzochinon in Acetonitril gelöst und vorgelegt. 0.8 g (1.5 mMol) der Silylverbindung, hergestellt gemäss Beispiel 3, werden in Acetonitril aufgenommen und bei einer Innentemperatur (IT) von 20-25°C zugetropft. Das Reaktionsgemisch wird 8 Stunden gerührt und über Silicagel gereinigt. Die schwach gefärbte klare Lösung wird bei AT 55-60°C eingeengt. Die resultierende Festsubstanz wird in Beispiel 4 eingesetzt.

### Beispiel 4 (Entfernen der Schutzgruppen und Kristallisation)

a) 0.5 g der Festsubstanz aus Beispiel 3 werden mit 20 g (0.175 Mol) Trifluoressigsäure versetzt und etwa 15 Stunden am Rückfluss erhitzt. Dabei wird die Trifluoressigsäure als Reagens und als Lösungsmittel eingesetzt. Nach dem Abkühlen wird das Reaktionsgemisch auf eine Mischung von 300 g gesättigter Natriumbikarbonatlösung und 50 g Eis gegossen und mit 20 g Essigsäureethylester extrahiert.
b) Das im vorgehenden Abschnitt a) erhaltene braune Rohprodukt wird in Toluol bei 90°C (Massenverhältnis Toluol : Rohware = 6:1) gelöst, auf 20-25°C abgekühlt. Die ausgefallene, grauweisse Masse wird bei 20-25°C abfiltriert und getrocknet. Man erhält Finasterid Polymorph I.

### Beispiel 5 (Einführung der Δ¹-Doppelbindung zu 4-Benzyloxycarbonyl-Finasterid)

2.0 g (3.7 mMol) der Verbindung aus Beispiel 2 werden mit 1.29 g (11.1 mMol) Allylmethylcarbonat in Acetonitril gemischt. Die Mischung wird zu einer 60-70°C heissen Lösung aus 166 mg (0.74 mMol) Palladium-II-acetat in Acetonitril zugetropft. Nach 1-2 Stunden am Rückfluss wird wie in Beispiel 3 beschrieben aufgearbeitet. Es werden 3 g Festsubstanz erhalten.

### Beispiel 6 (Einführung der Δ¹-Doppelbindung)

A) 20 g (0.047 mol) des Oxalylenolethers von Dihydrofinasterid [Verbindung IIIa, mit R= -NH-tert.Butyl, R₃ und R₄ = -C(O)-C(O)-] werden zusammen mit 16.3 g (0.140 mol) Allylmethylcarbonat und 76 g wasserfreiem Acetonitril auf Rückflusstemperatur erhitzt. Man gibt nacheinander 5 Portionen einer Mischung aus je 18g Xylol und je 0.049g Tris(dibenzylidenaceton)-diPalladium Chloroform-Komplex (totale Molmenge an Katalysator: 0.284 mmol) zu. Jedes Mal ist bei der Zugabe eine beträchtliche Gasentwicklung sichtbar. Nach 12 h Rückflussieren wird die Reaktion durch Zugabe zweier Portionen einer heissen Mischung aus je 3g Xylol und je 0.024g Dehydrierungskatalysator (Mischung langsam aufgeheizt) vervollständigt (falls nötig, werden weitere Portionen dazu geben). Die Reaktionsmischung wird nach der Filtration soweit wie möglich eingeengt, danach bleiben 24.5g einer gelben, honigartigen Masse zurück.
B) Die honigartige Masse wird in 105g Methanol aufgenommen und auf 0-5°C abgekühlt. Langsam dosiert man 11.3g (0.0403 mol) Kaliummethoxidlösung 25% zu und rührt ca. 1 Stunde bei 0-5°C Innentemperatur nach. Dann werden 20g Wasser zudosiert und das Kühlbad entfernt, die Innentemperatur steigt auf 15-20°C an. Die Mischung wird zur Trockene eingeengt, zum festen Rückstand 50g Wasser, 90g Toluol und 12g Methanol zugegeben und 1 Stunde auf Rückflusstemperatur erhitzt. Nach Abstellen des Rührers trennen sich organische Phase und Wasserphase problemlos; die organische Phase wird heiss abgetrennt. Das Abkühlen innert 2-4 Std. auf 25°C bringt das Finasterid in der polymorphen Form I zur Kristallisation. Nach dem Trocknen erhält man 8.1g weisses Pulver.

### Beispiel 7

In analoger Weise zu den in den Beispielen 1 bis 6 beschriebenen Verfahren geht man vor, wenn man die Δ¹-Doppelbindung in Dihydro-Dutasterid einführt, d.h. in eine entsprechende Dihydroverbindung der Formel (I), worin R einen Rest -NHR₁, und R₁ 2,5-Bis(trifluoromethyl)phenyl bedeuten, wobei durch die Einführung der Δ¹-Doppelbindung Dutasterid erhalten wird.

### Beispiel 8 (Herstellung von 3-Oxo-4-aza-5a-androst-1-en-17β-carbonsäuremethylester)

### Stufe 1 (Herstellung der Verbindung IIIb, d.i. eine Verbindung der Formel (III), worin R= -OMe, R₃ und R₄= -C(O)-C(O)-:

2g (0.005mol, Gehalt >95%) 3-Oxo-4-aza-5α-androst-1-en-17β-carbonsäuremethylester werden mit 30g Toluol versetzt und unter Kühlung langsam 2.6g (0.019 mol) Oxalylchlorid zugegeben. Allmählich setzt eine konstante Gasentwicklung ein. Die trübe Mischung wird über Nacht gerührt. Aus der klaren Reaktionslösung werden bei Raumtemperatur unter vermindertem Druck überschüssiges Oxalylchlorid und Toluol bis zur Hälfte des ursprünglichen Volumens destillativ entfernt. Dabei scheidet sich ein weisser Feststoff ab, der filtriert und dreimal mit je 15g Heptan intensiv gewaschen wird. Nach dem Trockensaugen bleiben 1.6g roher Methylester zurück. Dieser wird in ca. 20g Dichlormethan aufgenommen, die trübe Lösung mit 33g 5% Kaliumbicarbonatlösung intensiv gewaschen, die Mischung filtriert und die organische Phase dreimal mit je 10g Wasser nachgewaschen. Die klare, farblose organische Phase wird soweit möglich eingeengt und 0.9g der Verbindung IIIb erhalten.
¹H-NMR (200MHz, CDCl₃, δ): 4.95 (1H,t); 3.68 (3H,s); 3.62-3.5 (1H,m); 3.22-3.06 (1H,m); 2.41-0.80 (17H,m); 0.97(3H,s); 0.68 (3H,s)

### Stufe 2 (Einführung der Δ¹-Doppelbindung):

0.2g (0.5mmol) des in Stufe 1 hergestellten Verbindung IIIb werden zusammen mit 8g absolutem Acetonitril, 1.5g Chloroform, 0.18g (1.5mmol) Allylmethylcarbonat und 0.05g (0.05mol) Palladium-Katalysator auf Rückflusstemperatur (70-80°C) erhitzt. Bereits beim Aufheizen ist eine Gasentwicklung sichtbar. Nach ca. 30 Minuten Rückflussieren wird die Reaktionsmischung soweit möglich eingeengt, der Rückstand in einer Mischung aus 15g Methanol und 5g Toluol aufgenommen und erwärmt, bis eine klare Lösung vorliegt. Nach Abkühlen auf 0-5°C wird eine Lösung aus 0.18g (1 mmol) Natriummethylat-Lösung 30% in 2g Methanol langsam zudosiert und die klare Lösung 1 Std. gerührt. Nach Entfernen des Kühlbades werden 3g Wasser dazu gegeben und die trübe Mischung bei Raumtemperatur 1 Stunde nachgerührt. Danach wird soweit möglich eingeengt und 10g Toluol sowie 3g Wasser zum Rückstand gegeben. Sobald beim Erwärmen das Gemisch sich in zwei klare Phasen aufgetrennt hat, wird sofort die organische Phase abgetrennt und abgekühlt. Die Zugabe von 2-4g Heptan bringt das Produkt zur Kristallisation. Nach dem Filtrieren, Nachwaschen mit ca. 5g Heptan und Trockensaugen bleiben 34 mg 3-Oxo-4-aza-5α-androst-1-en-17β-carbonsäuremethylester zurück. ¹H-NMR (200MHz, CDCl₃, δ): 6.81 (1H,d); 5.82(1H,d); 5.48(1H,s breit); 3.69 (3H,s); 3.4-3.35 (1H,m); 2.45-1.0(17H,m); 0.97(3H,s); 0.66 (3H,s)

### Beispiel 9 (Herstellung von Dutasterid)

### Stufe 1 (Herstellung von 3-Oxo-4-aza-5α-androstan-17β-carbonsäure):

Eine Suspension aus 100g (0.26mol) Dihydrofinasterid, 480g 20% HCl-Lösung (2.63 mol) und 120g Methanol wird auf Rückfluss erhitzt und 8-12 Stunden intensiv gekocht. Das Edukt geht beim Erhitzen in Lösung, nach 8 Stunden liegt eine Suspension vor, die leicht filtriert werden kann. Der Nutschkuchen wird dreimal intensiv mit je 100g Wasser gewaschen, ca. 15 Minuten trockengesaugt und anschliessend über Nacht getrocknet. Ausbeute: 60g.
¹H-NMR (200MHz, DMSO, δ): 11.95 (1H, s); 7.32 (1H, s); 2.95 (1H, m); 2.2 (2H, m); 2.0 - 0.85 (17H, m); 0.81(3H, s); 0.62 (3H, s)

### Stufe 2 (Herstellung der Verbindung IIIc, d.i. eine Verbindung der Formel (III), worin R=Cl, R₃ und R₄= -C(O)-C(O)-:

Zu einer Suspension aus 40g (0.12 mol) der Verbindung aus Stufe 1 in 633g Benzol werden innert 20-30 Minuten unter Kühlung 159g (1.2 mol) Oxalylchlorid zugetropft und die Suspension 12h gerührt (keine Gasentwicklung mehr sichtbar). Unter vermindertem Druck werden bei Raumtemperatur Benzol und überschüssiges Oxalylchlorid solange destillativ abgetrennt, bis das Volumen der ursprünglichen Lösung auf die Hälfte reduziert ist. Dabei fällt ein grauweisser Feststoff aus, der nach der Filtration dreimal mit je 150g Heptan gewaschen und etwa 15 Minuten trockengesaugt wird. Ausbeute: 37.1g der Verbindung IIIc.
¹H-NMR (200MHz, CDCl₃, δ): 4.93 (1H, t); 3.58 (1H, m); 3.12 (1H, m); 2.88 (1H, m); 2.31-0.72 (18H, m); 0.97(3H, s); 0.80 (3H, s)

### Stufe 3 (Herstellung der Verbindung IIId (R= -NH-(2,5-(CF₃)₂-C₆H₃), R₃ und R₄ = -C(O)-C(O)-):

Eine Suspension aus 1.48g(6mmol) bis-2,5-Trifluormethylanilin, 2.35g (5.3 mmol) der Verbindung IIIc aus Stufe 2 und 50g Toluol wird ca. 8 Stunden auf Rückflusstemperatur (100-110°C) erhitzt und dann abgekühlt. Unter vermindertem Druck werden bei Raumtemperatur Toluol und Anilin solange destillativ abgetrennt, bis das Volumen der ursprünglichen Lösung auf die Hälfte reduziert ist. Zur Suspension gibt man 30g Heptan und erwärmt auf 60-70°C auf. Nach einer Stunde intensivem Rühren wird abgenutscht, der Nutschkuchen mit je 10g Heptan viermal intensiv gewaschen und ca. 30-45 Minuten trocken gesaugt. Ausbeute: 1.7g der Verbindung IIId.
¹H-NMR (200MHz, CDCl₃, δ): 8.79 (1H, s breit); 7.72 (1H, d); 7.49 (2H, m); 4.93 (1H, t); 3.59 (1H, m); 3.17 (1H, m); 2.38-1.0 (17H, m); 0.97(3H, s); 0.81 (3H, s)

### Stufe 4 (Herstellung von Dutasterid)

1g(1.6mmol) der Verbindung IIId aus Stufe 3 werden zusammen mit 8g absolutem Acetonitril, 2g Chloroform, 0.55g (4.8mol) Allylmethylcarbonat und 0.17g (0.16mol) Palladium-Katalysator auf Rückflusstemperatur (70-80°C) erhitzt. Bereits beim Erwärmen ist eine Gasentwicklung sichtbar. Nach ca. 30 Minuten Rückflussieren (keine Gasentwicklung mehr sichtbar) wird die Reaktionsmischung soweit möglich eingeengt und der Rückstand in 5g Methanol aufgenommen. Nach Abkühlen auf 0-5°C wird eine Lösung aus 0.6g (3.2 mmol) Natriummethylat-Lösung 30% in 4g Methanol langsam zudosiert und die klare Lösung 1 Stunde ebenfalls bei 0-5°C Innentemperatur gerührt. Nach Entfernen des Kühlbades werden 3g Wasser dazu gegeben, bei Raumtemperatur 1 Stunde nachgerührt, die trübe Mischung soweit als möglich eingeengt und 20g Toluol sowie 6g Wasser zum Rückstand gegeben. Die Mischung wird auf Rückflusstemperatur erhitzt. Nach 30 Minuten wird die klare organische Phase heiss abgetrennt und auf Raumtemperatur abgekühlt. Die Zugabe von 5-10g Heptan bringt das Dutasterid zur Kristallisation. Nach dem Filtrieren, dreimaligem Nachwaschen mit je 4g Heptan und Trockensaugen bleiben 0.3g Dutasterid zurück.
¹H-NMR (200MHz, CDCl₃, δ): 8.80 (1H, s breit); 7.75 (1H, d); 7.49 (2H, m); 6.80 (1H, d); 5.82 (1H, d); 8.80 (1H, s breit); 5.46 (1H, s breit); 3.35 (1H, m); 2.38-1.0 (17H, m); 0.97(3H, s); 0.81 (3H, s)

## Patentansprüche

1. Verfahren zur Herstellung von 17β-substituierten 4-Aza-androst-1-en-3-on-Verbindungen der allgemeinen Formel (I): 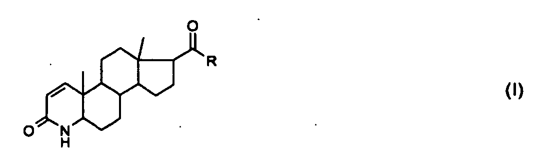 worin
R Hydroxyl, gegebenenfalls substituiertes, lineares oder verzweigtes (C₁-C₁₂)-Alkyl oder (C₁-C₁₂)-Alkenyl; Phenyl oder Benzyl; einen Rest -OR₁, oder einen Rest -NHR₁, oder einen Rest -NR₁R₂;
R₁ Wasserstoff, gegebenenfalls substituiertes, lineares oder verzweigtes (C₁-C₁₂)-Alkyl oder (C₁-C₁₂)-Alkenyl, oder gegebenenfalls substituiertes Phenyl;
R₂ Wasserstoff, Methyl, Ethyl oder Propyl; oder
-NR₁R₂ einen 5- oder 6-gliedrigen heterocyclischen Ring, und für R = Hydroxyl auch ein pharmazeutisch zugelassenes Salz davon,
bedeuten, **dadurch gekennzeichnet, dass** man
(A) in die 3-Keto-4-aza-Gruppierung, d.h. die Lactamgruppierung, einer Verbindung der allgemeinen Formel (II): 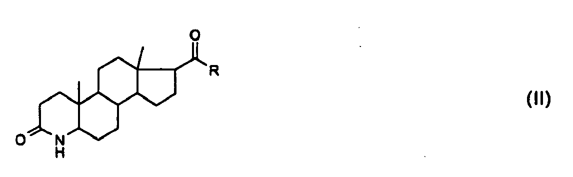 Schutzgruppen einführt, so dass eine Verbindung der allgemeinen Formel (III) entsteht: 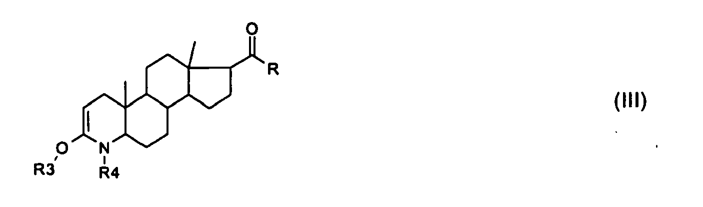 worin
R₃ Trialkylsilyl, oder zusammen mit R₄ den Rest -C(O)-C(O)- oder -C(O)-Y-C(O)-;
R₄ Alkyloxycarbonyl oder Phenyloxycarbonyl, vorzugsweise Boc, = tert.-Butyloxycarbonyl; oder Trialkylsilyl, oder zusammen mit R₃ den Rest -C(O)-C(O)- oder -C(O)-Y-C(O)-;
Y -[C(R₅)(R₆)]ₙ-, oder -CH(R₅)=CH(R₆)-, oder ortho- Phenylen;
R₅ und R₆ unabhängig voneinander Wasserstoff, lineares oder verzweigtes (C₁₋₈)-Alkyl oder Alkenyl, gegebenenfalls substituiertes Phenyl oder Benzyl; und
n eine ganze Zahl von 1 bis 4,
bedeuten;
und worin für den Fall, dass R Hydroxyl bedeutet, diese gegebenenfalls mit einer Schutzgruppe reagiert hat;
(B) die [gemäss Schritt (A)] erhaltene Verbindung in Gegenwart (i) eines Dehydrierungskatalysators, welcher ausgewählt ist aus Verbindungen von Eisen, Ruthenium und Osmium; Cobalt, Rhodium und Iridium; Nickel, Palladium und Platin; Kupfer, Silber und Gold und in Gegenwart von (ii) gegebenenfalls substituiertem Benzochinon, Allylmethylcarbonat, Allylethylcarbonat und/oder Allylpropylcarbonat, umsetzt, wobei die Δ¹-Doppelbindung in 1-/2-Stellung eingeführt wird, und
(C) die Schutzgruppen R₃ und R₄ entfernt und für R = Hydroxyl, die erhaltene Verbindung gegebenenfalls in ein Salz umwandelt.

2. Verfahren nach Anspruch 1, **dadurch gekennzeichnet, dass** R lineares oder verzweigtes (C₁-C₆)-Alkyl, vorzugsweise Methyl, Ethyl, Propyl oder n-Butyl, sec.-Butyl oder tert.-Butyl, vorzugsweise tert.-Butyl; oder einen Rest -OR₁, oder ein Rest -NHR₁, oder ein Rest -NR₁R₂, vorzugsweise einen Rest -NHR₁, vorzugsweise -NH-tert.-Butyl, oder gegebenenfalls substituiertes Phenyl, bedeutet.

3. Verfahren nach Anspruch 1 oder 2, **dadurch gekennzeichnet, dass** R₁ lineares oder verzweigtes (C₁-C₆)-Alkyl, vorzugsweise Methyl, Ethyl, Propyl, n-Butyl, sec.-Butyl oder tert.-Butyl, vorzugsweise tert.-Butyl, bedeutet.

4. Verfahren nach Anspruch 1 oder 2, **dadurch gekennzeichnet, dass** R eines Rest -NHR₁ bedeutet, worin R₁ 2,5-Bis(trifluoromethyl)phenyl bedeutet.

5. Verfahren nach Anspruch 1 oder 2, **dadurch gekennzeichnet, dass** im Rest -NR₁R₂ der Substituent R₂ Methyl bedeutet.

6. Verfahren nach Anspruch 1 oder 2, **dadurch gekennzeichnet, dass** der Substituent -NR₁R₂ als 5- oder 6-gliedriger heterocyclischer Ring einen Rest von Piperidin oder Pyrrolidin bedeutet.

7. Verfahren nach einem der Ansprüche 1-6, **dadurch gekennzeichnet, dass** R₃ Trimethylsilyl, oder zusammen mit R₄ den Rest -C(O)-C(O)- oder -C(O)-Y-C(O)- bedeutet.

8. Verfahren nach einem der Ansprüche 1-6, **dadurch gekennzeichnet, dass** R₄ Alkyloxycarbonyl, vorzugsweise Isobutyloxycarbonyl, tert.-Butyloxycarbonyl, tert.-Amyloxycarbonyl, Cyclobutyloxycarbonyl, 1-Methylcylobutyloxycarbonyl, Cyclopentyloxycarbonyl, Cyclohexyloxycarbonyl, 1-Methylcyclohexyloxycarbonyl, vorzugsweise tert.-Butyloxycarbonyl, bedeutet.

9. Verfahren nach einem der Ansprüche 1-8, **dadurch gekennzeichnet, dass** R₄ Boc, Trimethylsilyl, oder zusammen mit R₃ den Rest -C(O)-C(O)- oder -C(O)-Y-C(O)-, vorzugsweise Boc oder zusammen mit R₃ den Rest -C(O)-C(O)- oder -C(O)-Y-C(O)-, bedeutet.

10. Verfahren nach Anspruch 9, **dadurch gekennzeichnet, dass** R₅ und R₆ unabhängig voneinander Wasserstoff, lineares oder verzweigtes (C₁₋₄)-Alkyl, oder Phenyl, vorzugsweise Wasserstoff, Methyl, Ethyl oder Propyl oder Phenyl, vorzugsweise den Rest -CH(R₅)- oder ortho-Phenylen, vorzugsweise Methylen, und n 1 oder 2, vorzugsweise 1, bedeuten.

11. Verfahren nach einem der Ansprüche 1-9, **dadurch gekennzeichnet, dass** man für die Einführung der Schutzgruppe Boc die Verbindung der allgemeinen Formel (II) Boc-Anhydrid oder Boc-Carbamat oder eine analoge Verbindung verwendet, worin der tert.-Butylrest ersetzt ist durch tert.-Amyl, Cyclobutyl, Cyclopentyl oder Cyclohexyl.

12. Verfahren nach einem der Ansprüche 1-11, **dadurch gekennzeichnet, dass** der Dehydrierungskatalysator im Schritt (B) ausgewählt ist aus Verbindungen auf der Basis von Rhodium, Palladium und Platin.

13. Verfahren nach Anspruch 12, **dadurch gekennzeichnet, dass** der Dehydrierungskatalysator im Schritt (B) ausgewählt ist aus Pd(0)-Verbindungen, und vorzugsweise den Tris(dibenzylidenaceton)diPalladium-Chloroform Komplex darstellt.

14. Verfahren nach Anspruch 12, **dadurch gekennzeichnet, dass** der Dehydrierungskatalysator [im Schritt B)] ausgewählt ist aus Pd(II)-Verbindungen, vorzugsweise aus PdCl₂, Pd(dppe)₂, worin dppe = bis-(1,2-biphenylphosphino)ethan, Pd(dppe)Cl₂, Pd(OAc)₂, Pd(dppe)(OAc)₂ und/oder aus π-Allyl-Pd-Komplexe, vorzugsweise π-Allyl-Pd-chlorid Dimer.

15. Verfahren nach einem der Ansprüche 1-14, **dadurch gekennzeichnet, dass** zur termischen Stabilisierung des Dehydrierungskatalysators, vorzugsweise des Palladiumsalzes oder des Palladiumkomplexes ein zusätzlicher Komplexbildner, vorzugsweise 2,2'-Bipyridyl oder 1,10-Phenanthrolin, vorzugsweise 2,2'-Bipyridyl, anwesend ist.

16. Verfahren nach einem der Ansprüche 1-15, **dadurch gekennzeichnet, dass** man als Chinon in Schritt (B) ein substituiertes Chinon verwendet, vorzugsweise ein durch C₁₋₄-Alkyl, Halogen, Cyano oder Nitro substituiertes Chinon.

17. Verfahren nach einem der Ansprüche 1-16, **dadurch gekennzeichnet, dass** man in Schritt (C) die eingeführten Schutzgruppen durch Behandlung mit einer geeigneten Säure, vorzugsweise durch Behandlung mit Ameisensäure, Essigsäure und/oder Trifluoressigsäure, vorzugsweise mit Ameisensäure, entfernt.

18. Verfahren nach einem der Ansprüche 1-17, **dadurch gekennzeichnet, dass** man in Schritt (C) die erhaltene Verbindung, worin R Hydroxyl bedeutet, in ein Alkalisalz, ein Erdalkalisalz oder ein Ammoniumsalz, vorzugsweise in ein Salz von Natrium, Kalium oder Ammonium, vorzugsweise in ein Salz von Natrium oder Kalium, umwandelt.

19. Verfahren nach einem der Ansprüche 1-17, **dadurch gekennzeichnet, dass** man die erhaltene Verbindung der Formel (I) aus einem apolaren Lösungsmittel, vorzugsweise aus Benzin, Heptan, Hexan und/oder Toluol, vorzugsweise aus Toluol, kristallisiert.

20. Verfahren nach Anspruch 19, **dadurch gekennzeichnet, dass** man die erhaltene Verbindung der Formel (I), welche 17β-(N-tert.-Butylcarbamoyl)-4-aza-androst-1-en-3-on darstellt, aus einer gesättigten Lösung aus Toluol bei einer Temperatur von etwa 25°C in der polymorphen Form I kristallisiert.

21. Verfahren nach Anspruch 19, **dadurch gekennzeichnet, dass** man die erhaltene Verbindung der Formel (I), welche 17β-(N-tert.-Butylcarbamoyl)-4-aza-androst-1-en-3-on darstellt, aus einer gesättigten Lösung aus Toluol bei einer Temperatur von etwa 0°C in der polymorphen Form II kristallisiert.

## Claims

1. Process for the preparation of 17β-substituted 4-azaandrost-1-en-3-one compounds of general formula (I): 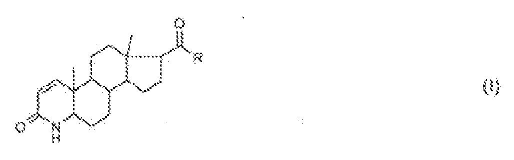 in which
R is hydroxyl, optionally substituted, linear or branched (C₁-C₁₂)-alkyl or (C₁-C₁₂)-alkenyl; phenyl or benzyl; a radical -OR₁, a radical -NHR₁ or a radical -NR₁R₂;
R₁ is hydrogen, optionally substituted, linear or branched (C₁-C₁₂)-alkyl or (C₁-C₁₂)-alkenyl, or optionally substituted phenyl; and
R₂ is hydrogen, methyl, ethyl or propyl; or
-NR₁R₂ is a 5- or 6-membered heterocyclic ring, and, in the case where R = hydroxyl, also a pharmaceutically acceptable salt thereof,
**characterized in that**
(A) protecting groups are introduced into the 3-keto-4-aza grouping, i.e. the lactam grouping, of a compound of general formula (II): 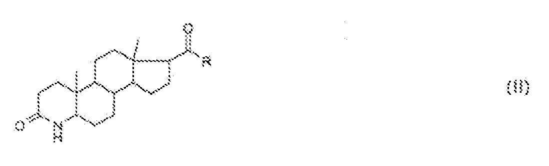 to give a compound of general formula (III): 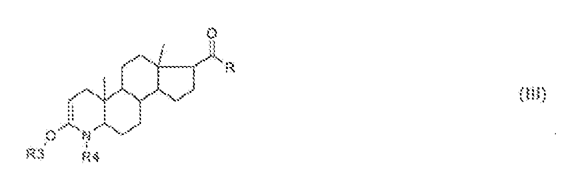 in which
R₃ is trialkylsilyl, or together with R₄ the radical -C(O)-C(O)- or -C(O)-Y-C(O)-;
R₄ is alkoxycarbonyl or phenoxycarbonyl, preferably Boc (= tert-butoxycarbonyl); or trialkylsilyl, or together with R₃ the radical -C(O)-C(O)- or -C(O)-Y-C(O)-;
Y is -[C(R₅)(R₆)]ₙ-, -CH(R₅)=CH(R₆)- or orthophenylene;
R₅ and R₆ independently of one another are hydrogen, linear or branched (C₁-C₈)-alkyl or alkenyl, or optionally substituted phenyl or benzyl; and
n is an integer from 1 to 4,
and in which, in the case where R is hydroxyl, this may have reacted with a protecting group;
(B) the compound obtained [according to step (A)] is reacted in the presence of (i) a dehydrogenation catalyst selected from compounds of iron, ruthenium and osmium; cobalt, rhodium and iridium; nickel, palladium and platinum; and copper, silver and gold, and in the presence of (ii) optionally substituted benzoquinone, allyl methyl carbonate, allyl ethyl carbonate and/or allyl propyl carbonate, the Δ¹ double bond being introduced into the 1,2-position; and
(C) the protecting groups R₃ and R₄ are removed and, in the case where R = hydroxyl, the compound obtained is optionally converted to a salt.

2. Process according to Claim 1, **characterized in that** R is linear or branched (C₁-C₆)-alkyl, preferably methyl, ethyl, propyl, n-butyl, sec-butyl or tert-butyl, preferably tert-butyl; or a radical -OR₁, a radical -NHR₁ or a radical -NR₁R₂, preferably a radical -NHR₁, preferably -NH-tert-butyl; or optionally substituted phenyl.

3. Process according to Claim 1 or 2, **characterized in that** R₁ is linear or branched (C₁-C₆)-alkyl, preferably methyl, ethyl, propyl, n-butyl, sec-butyl or tert-butyl, preferably tert-butyl.

4. Process according to Claim 1 or 2, **characterized in that** R is a radical -NHR₁ in which R₁ is 2,5-bis(trifluoromethyl)phenyl.

5. Process according to Claim 1 or 2, **characterized in that**, in the radical -NR₁R₂, the substituent R₂ is methyl.

6. Process according to Claim 1 or 2, **characterized in that** the substituent -NR₁R₂ as a 5- or 6-membered heterocyclic ring is a piperidine or pyrrolidine radical.

7. Process according to one of Claims 1-6, **characterized in that** R₃ is trimethylsilyl, or together with R₄ the radical -C(O)-C(O)- or -C(O)-Y-C(O)-.

8. Process according to one of Claims 1-6, **characterized in that** R₄ is alkoxycarbonyl, preferably isobutoxycarbonyl, tert-butoxycarbonyl, tert-amyloxycarbonyl, cyclobutoxycarbonyl, 1-methylcyclobutoxycarbonyl, cyclopentoxycarbonyl, cyclohexyloxycarbonyl or 1-methylcyclohexyloxycarbonyl, preferably tert-butoxycarbonyl.

9. Process according to one of Claims 1-8, **characterized in that** R₄ is Boc, trimethylsilyl, or together with R₃ the radical -C(O)-C(O)- or -C(O)-Y-C(O)-, preferably Boc or together with R₃ the radical -C(O)-C(O)- or -C(O)-Y-C(O)-.

10. Process according to Claim 9, **characterized in that** R₅ and R₆ independently of one another are hydrogen, linear or branched (C₁-C₄)-alkyl or phenyl, preferably hydrogen, methyl, ethyl, propyl or phenyl, is preferably the radical -CH(R₅)- or orthophenylene, preferably methylene, and n is 1 or 2, preferably 1.

11. Process according to one of Claims 1-9, **characterized in that** the protecting group Boc is introduced the compound of general formula (II) using Boc anhydride, Boc carbamate or an analogous compound in which the tert-butyl radical has been replaced by tert-amyl, cyclobutyl, cyclopentyl or cyclohexyl.

12. Process according to one of Claims 1-11, **characterized in that** the dehydrogenation catalyst in step (B) is selected from compounds based on rhodium, palladium and platinum.

13. Process according to Claim 12, **characterized in that** the dehydrogenation catalyst in step (B) is selected from Pd(0) compounds and is preferably the tris-(dibenzylideneacetone)dipalladium-chloroform complex.

14. Process according to Claim 12, **characterized in that** the dehydrogenation catalyst in step (B) is selected from Pd(II) compounds, preferably PdCl₂, Pd(dppe)₂, in which dppe = bis(1,2-biphenylphosphino)ethane, Pd(dppe)Cl₂, Pd(OAc)₂ and Pd(dppe)(OAc)₂, and/or from n-allyl-Pd complexes, preferably π-allyl-Pd chloride dimer.

15. Process according to one of Claims 1-14, **characterized in that** an additional complexing agent, preferably 2,2'-bipyridyl or 1,10-phenanthroline, preferably 2,2'-bipyridyl, is present for thermal stabilization of the dehydrogenation catalyst, preferably the palladium salt or the palladium complex.

16. Process according to one of Claims 1-15, **characterized in that** the quinone used in step (B) is a substituted quinone, preferably a quinone substituted by C₁₋₄-alkyl, halogen, cyano or nitro.

17. Process according to one of Claims 1-16, **characterized in that**, in step (C), the protecting groups introduced are removed by treatment with a suitable acid, preferably by treatment with formic acid, acetic acid and/or trifluoroacetic acid, preferably formic acid.

18. Process according to one of Claims 1-17, **characterized in that**, in step (C), the compound obtained in which R is hydroxyl is converted to an alkali metal salt, an alkaline earth metal salt or an ammonium salt, preferably a sodium, potassium or ammonium salt, preferably a sodium or potassium salt.

19. Process according to one of Claims 1-17, **characterized in that** the compound of formula (I) obtained is crystallized from an apolar solvent, preferably naphtha, heptane, hexane and/or toluene, preferably toluene.

20. Process according to Claim 19, **characterized in that** the compound of formula (I) obtained is 17β-(N-tert-butylcarbamoyl)-4-azaandrost-1-en-3-one and is crystallized in polymorphous form I from a saturated solution of toluene at a temperature of about 25°C.

21. Process according to Claim 19, **characterized in that** the compound of formula (I) obtained is 17β-(N-tert-butylcarbamoyl)-4-azaandrost-1-en-3-one and is crystallized in polymorphous form II from a saturated solution of toluene at a temperature of about 0°C.

## Revendications

1. Procédé pour la fabrication de composés 4-aza-androst-1-en-3-one 17β-substitués de formule générale (I) : 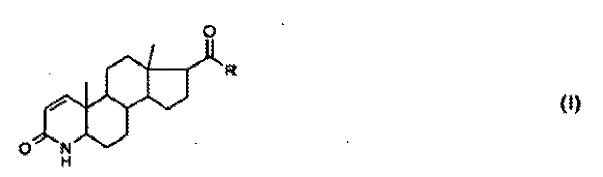 dans laquelle
R représente un hydroxyle, optionnellement un (C₁-C₁₂) alkyle ou (C₁-C₁₂) alcényle substitué, linéaire ou ramifié ; un phényle ou benzyle, un reste -OR₁, ou un reste - NHR₁, ou un reste -NR₁R₂ ;
R₁ représente un hydrogène, optionnellement un (C₁-C₁₂) alkyle ou (C₁-C₁₂) alcényle substitué, linéaire ou ramifié, ou optionnellement un phényle substitué ;
R₂ représente un hydrogène, méthyle, éthyle ou propyle ; ou -NR₁R₂ un anneau hétérocyclique à 5 ou 6 membres, et pour R = hydroxyle également un sel pharmaceutiquement acceptable de celui-ci,
**caractérisé en ce que** l'on
(A) introduit dans le groupement 3-keto-4-aza, c'est-à-dire le groupement de lactame, d'un composé de formule générale (II) : 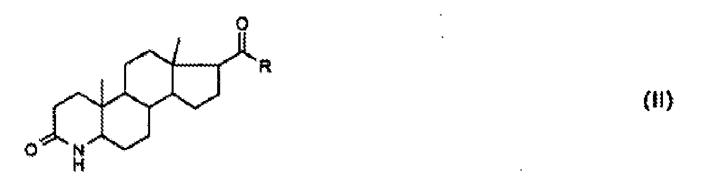 des groupes protecteurs de telle manière qu'un composé de formule générale (III) se forme : 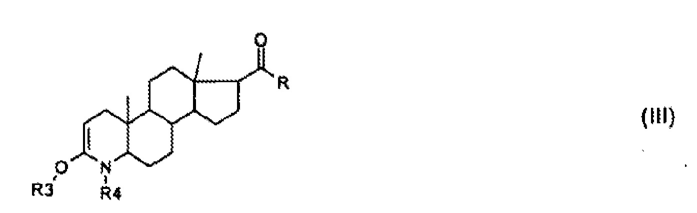 dans laquelle
R₃ représente un trialkylsilyle, ou ensemble avec R₄ le reste -C(O)-C(O)- ou -C(O)-Y-C(O)-;
R₄ représente un alkyloxycarbonyle ou phényloxycarbonyle, de préférence unBoc, tert- butyloxycarbonyle ; ou trialkylsilyle, ou ensemble avec R₃ le reste -C(O)-C(O)- ou -C(O)-Y-C(O)- ;
Y représente -[C(R₅)(R₆)]ₙ-, ou -CH(R₅)=CH(R₆)-, ou un ortho-phénylène ;
R₅ et R₆, indépendamment l'un de l'autre, représentent un hydrogène, (C₁-C₈) alkyle ou alcényle linéaire ou ramifié, optionnellement un phényle ou benzyle substitué ; et
n représente un nombre entier de 1 à 4,
et où dans le cas où R représente un hydroxyle, celui-ci a optionnellement réagi avec un groupe protecteur ;
(B) réagit le composé obtenu [selon l'étape (A)] en présence (i) d'un catalyseur de déshydratation qui est choisi parmi les composés de fer, ruthénium et osmium ; cobalt, rhodium et iridium ; nickel, palladium et platine ; cuivre, argent et or et en présence (ii) optionnellement de benzoquinone substituée, allylméthylcarbonate, allyléthylcarbonate et/ ou allylpropylcarbonate, où la double liaison Δ¹ est introduite en position 1/ 2, et
(C) élimine les groupes protecteurs R₃ et R₄ et pour R = hydroxyle, transforme le composé obtenu optionnellement en un sel.

2. Procédé selon la revendication 1, **caractérisé en ce que** R représente un (C₁-C₆) alkyle linéaire ou ramifié, de préférence un méthyle, éthyle, propyle ou n-butyle, sec-butyle ou tert-butyle, de préférence un tert-butyle ; ou un reste -OR₁, ou un reste -NHR₁, ou un reste -NR₁R₂, de préférence un reste -NHR₁, de préférence un -NH-tert-butyle, ou optionnellement un phényle substitué.

3. Procédé selon la revendication 1 ou 2, **caractérisé en ce que** R₁ représente un (C₁-C₆) alkyle linéaire ou ramifié, de préférence un méthyle, éthyle, propyle, n-butyle, sec-butyle ou tert-butyle, de préférence un tert-butyle.

4. Procédé selon la revendication 1 ou 2, **caractérisé en ce que** R représente un reste -NHR₁, où R₁ représente un 2,5-Bis(trifluorométhyl)phényle.

5. Procédé selon la revendication 1 ou 2, **caractérisé en ce que** dans le reste -NR₁R₂ le substituant R₂ représente un méthyle.

6. Procédé selon la revendication 1 ou 2, **caractérisé en ce que** le substituant -NR₁R₂ représente un reste de pipéridine ou de pyrrolidine sous forme d'un anneau hétérocyclique à 5 ou 6 membres.

7. Procédé selon l'une des revendications 1 à 6, **caractérisé en ce que** R₃ représente un triméthylsilyle, ou ensemble avec R₄ le reste -C(O)-C(O)- ou -C(O)-Y-C(O)-.

8. Procédé selon l'une des revendications 1 à 6, **caractérisé en ce que** R₄ représente un alkyloxycarbonyle, de préférence un isobutyle-oxycarbonyle, tert-butyloxycarbonyle, tert-amyloxycarbonyle, cyclobutyloxycarbonyle, 1-méthylcyclobutyloxycarbonyle, cyclopentyloxycarbonyle, cyclohexyloxycarbonyle, 1-méthylcyclohexyloxycarbonyle, de préférence un tert-butyloxycarbonyle.

9. Procédé selon l'une des revendications 1 à 8, **caractérisé en ce que** R₄ représente un Boc, triméthylsilyle, ou ensemble avec R₃ le reste -C(O) - C(O) - ou -C(O)-Y-C(O)-, de préférence un Boc ou ensemble avec R₃ le reste -C(O)-C(O) - ou -C(O)-Y-C(O)-.

10. Procédé selon la revendication 9, **caractérisé en ce que** R₅ et R₆ indépendamment l'un de l'autre représentent un hydrogène, (C₁-C₄) alkyle linéaire ou ramifié, ou phényle, de préférence un hydrogène, méthyle, éthyle ou propyle ou phényle, de préférence le reste - CH(R₅)- ou ortho-phénylène, de préférence un méthylène, et n représente 1 ou 2, de préférence 1.

11. Procédé selon l'une des revendications 1 à 9, **caractérisé en ce que** pour l'introduction du groupe protecteur Boc est utilisé le composé de formule générale (II) Boc-anhydride ou Boc-carbamate ou un composé analogue, où le reste tert-butyle est remplacé par un tert-amyle, cyclobutyle, cyclopentyle ou cyclohexyle.

12. Procédé selon l'une des revendications 1 à 11, **caractérisé en ce que** le catalyseur de déshydratation dans l'étape (B) est choisi parmi les composés sur la base de rhodium, palladium et platine.

13. Procédé selon la revendication 12, **caractérisé en ce que** le catalyseur de déshydratation dans l'étape (B) est choisi parmi les composés de Pd(0), et représente de préférence le complexe tris(dibenzylidenacétone)dipalladium-chloroforme.

14. Procédé selon la revendication 12, **caractérisé en ce que** le catalyseur de déshydratation [dans l'étape (B)] est choisi parmi les composés de Pd(II), de préférence de PdCl₂, Pd(dppe)₂, où dppe = bis-(1,2-biphénylphosphino)éthane, Pd(dppe)Cl₂, Pd(OAc)₂, Pd(dppe) (OAc)₂ et/ ou les complexes π-allyl-Pd, de préférence le dimère π-allyl-Pd-chloride.

15. Procédé selon l'une des revendications 1 à 14, **caractérisé en ce que** pour la stabilisation thermique du catalyseur de déshydratation, de préférence un sel de palladium ou un complexe de palladium, un agent complexant additionnel est présent, de préférence 2,2'-bipyridyle ou 1,10-phénanthroline, de préférence 2,2'-bipyridyle.

16. Procédé selon l'une des revendications 1 à 15, **caractérisé en ce qu'**une quinone substituée est utilisée en tant que quinone dans l'étape (B), de préférence une quinone substituée par un C₁₋₄ alkyle, halogène, cyano ou nitro.

17. Procédé selon l'une des revendications 1 à 16, **caractérisé en ce que** dans l'étape (C) les groupes protecteurs introduits sont éliminés par le traitement avec un acide approprié, de préférence par le traitement avec de l'acide formique, acide acétique et/ ou acide trifluoracétique, de préférence avec de l'acide formique.

18. Procédé selon l'une des revendications 1 à 17, **caractérisé en ce que** dans l'étape (C) le composé obtenu, dans lequel R représente un hydroxyle, est transformé en un sel alcalin, un sel alcalinoterreux ou un sel d'ammonium, de préférence en un sel de sodium, potassium ou ammonium, de préférence en un sel de sodium ou potassium.

19. Procédé selon l'une des revendications 1 à 17, **caractérisé en ce que** l'on cristallise le composé de formule (I) obtenu à partir d'un solvant apolaire, de préférence de benzène, heptane, hexane et/ ou toluène, de préférence de toluène.

20. Procédé selon la revendication 19, **caractérisé en ce que** l'on cristallise le composé de formule (I) obtenu, qui représente une 17β-(N-tert-butylcarbamoyl)-4-aza-androst-1-en-3-one, à partir d'une solution saturée de toluène à une température d'environ 25°C dans la forme polymorphe I.

21. Procédé selon la revendication 19, **caractérisé en ce que** l'on cristallise le composé de formule (I) obtenu, qui représente une 17β-(N-tert-butylcarbamoyl)-4-aza-androst-1-en-3-one, à partir d'une solution saturée de toluène à une température d'environ 0°C dans la forme polymorphe II.
